# EUROPEAN PATENT SPECIFICATION

(11) **EP 1 788 019 B1**
(45) Date of publication and mention of the grant of the patent: **22.04.2009**
(21) Application number: 06023746.8
(22) Date of filing: 15.11.2006
(51) Int. Cl.: C08K 7/00, C08K 9/00, C08K 7/24, C08K 9/08

(54) **Nanotube composite and preparation thereof**
Nanoröhrenverbundwerkstoff und Verfahren zu seiner Herstellung
Matériau composite comprenant de nanotubes et procédé de son préparation

(30) Priority: 16.11.2005 KR 20050109526
(43) Date of publication of application: 23.05.2007
(73) Proprietor: Gwangju Institute of Science and Technology, Puk-gu Gwangju 500-712 (KR)
(72) Inventor: Geckeler, K.E., Puk-gu Gwangju 500-712 (KR); Nepal, Dhriti, Puk-gu Gwangju 500-712 (KR)
(74) Representative: Grünecker, Kinkeldey, Stockmair & Schwanhäusser Anwaltssozietät

(56) References cited:
- EP-A- 1 612 187
- WO-A-20/04097853
- WO-A-20/05100466
- US-A1- 2003 170 167
- US-A1- 2004 110 128

## Description

### FIELD OF THE INVENTION

The present invention relates to single-wall carbon nanotube-egg white protein composite (EW-SWNT), an aqueous dispersion comprising the same and preparation method thereof.

### BACKGROUND OF THE INVENTION

Single-wall carbon nanotube (SWNT) is one of the most fascinating materials due to its inimitable structural, mechanical and electronic properties and its aqueous dispersion has been become a subject of special interests in the field of biochemistry and biomedical science. A mono-dispersed solution of short and thin nanotubes having controlled interfacial properties is particularly sought after for application in the field of nanotube-polymer composite devices.

SWNTs exist in aggregated or bundled states due to their axial geometry and tube-tube van der Waals interactions, making it difficult to dissolve or disperse them in most solvents.

Both covalent and non-covalent methods have been used to debundle the nanotubes. The covalent method, however, destroys the intrinsic optical properties of the nanotubes. The non-covalent method for making individually dispersed SWNTs, on the other hand, allows the nanotubes to retain their most desirable electronic structure, and the use of various materials, such as synthesized polymers, surfactant, artificial DNA sequence and biopolymers such as specific peptide and DNA have been attempted.

In particular, EP 1 612 187 discloses that carbon nanotubes can be dispersed by sonication, the addition of surfactants, or DNA association. WO 2005/100466 discloses the use of poly(aryleneethynylene) polymers for exfoliating and dispersing/solubilizing nanomaterials such as single-walled carbon nanotubes (SWNTs). US 2003/170167 discloses the use of suitable dispersal agents, such as synthetic and natural detergents, deoxycholates, cyclodextrins, chaotropic salts and ion pairing agents in a method for isolating and purifying SWNTs from contaminants. WO 2004/097853 discloses the suspension of carbon nanotubes in a stabilizer, such as a surfactant or a water-soluble polymer, in a method for preparing a conductive carbon nanotube-polymer composite.

Although significant progresses have been achieved, there still remains a need to develop a simple and effective technique for a large-scale production of individually isolated SWNTs.

### SUMMARY OF THE INVENTION

Accordingly, it is an object of the present invention to provide a composite containing highly dispersed and debundlized single-wall carbon nanotubes (SWNTs).

It is another object of the present invention to provide an aqueous dispersion comprising the composite.

It is a further object of the present invention to provide a simple method for producing the composite.

It is a still further object of the present invention to provide a device comprising the composite or the aqueous dispersion.

In accordance with one aspect of the present invention, there is provided a single-wall carbon nanotube-egg white protein composite (EW-SWNT) comprising single-wall carbon nanotubes having non-covalent bonds with the egg white (EW) protein.

In accordance with another aspect of the present invention, there is provided an aqueous dispersion comprising the EW-SWNT.

In accordance with a further aspect of the present invention, there is provided a method for preparing the EW-SWNT which comprises the steps of homogenizing SWNTs in an aqueous solution of EW protein and removing solid bodies from the resulting homogenate.

In accordance with a still further aspect of the present invention, there is provided a device comprising the EW-SWNT.

### BRIEF DESCRIPTION OF THE DRAWINGS

The above and other objects and features of the present invention will become apparent from the following description of the invention, when taken in conjunction with the accompanying drawings, which respectively show:
Fig. 1: Photographs of vials containing (a) an aqueous solution of EW protein, (b) the inventive EW-SWNT dispersion and (c) a dispersion of only SWNTs in water;
Fig. 2: Graph representing UV/VIS absorbance spectra of the inventive EW-SWNT dispersion in water;
Fig. 3: Photo-luminescence spectra of the inventive EW-SWNT dispersion in D₂O solution; excitation was at 523 nm.
Fig. 4: Transmission electron microscope (TEM) scan of the SWNTs dispersion;
Fig. 5: Transmission electron microscope (TEM) scan of the inventive EW-SWNT dispersion.

### DETAILED DESCRIPTION OF THE INVENTION

The inventive single-wall carbon nanotube-egg white protein composite (EW-SWNT) has excellent solubility and dispersibility in water due to strong affinity between egg white (EW) protein and the surface of single-wall carbon nanotubes (SWNTs). The EW SWNT is stable in an aqueous solution for several months.

The EW protein, an amphoteric molecule, has both hydrophilic and hydrophobic domains, and their solubility and dispersibility depend on their amino acid sequence. The EW protein used in the present invention contains over 40 different proteins and attaches to the surface of nanotubes through coulomb interaction of the hydrophobic domains.

Further, the EW protein has antibiotic and metalphilic properties which are useful in the preparation of novel SWNT type nanotube-based biomedical devices. Moreover, electric charges of the EW protein changes depending on a pH or ion strength, and accordingly, the EW-SWNT, or an aqueous dispersion comprising the same can be advantageously employed in making various devices.

The inventive EW-SWNT can be prepared by a method which comprises the steps of homogenizing SWNTs in an aqueous solution of EW protein and removing solid bodies from the resulting homogenate.

The homogenization step may be conducted by subjecting the mixture of SWNTs and aqueous solution of EW protein to ultrasonication, e.g., for 30 minutes. The SWNTs may have a length in the range of 500 nm to 2 µm and be used in amount in the range of 3 to 10 weight% based on the EW protein.

The step of removing solid materials from the homogenized mixture may be conducted by any conventional methods, e.g., filtration, decating, ultracentrifugation, preferably, ultracentrifugation. The ultracentrifugation may be conducted at 10,000g to 200,000g, changing the rotational frequency gradually from low to high. For instance, the ultracentrifugation may be conducted in two stages, at 18,000g for 3 hours, followed by 120,000g for 4 hours.

The following Example is intended to further illustrate the present invention without limiting its scope.

### Example

Freeze-dried EW was dissolved in water to obtain an aqueous solution of EW protein. 3 mg of SWNTs were mixed with the solution (1 mg/Mℓ) and the resulting mixture was subjected to ultrasonic treatment at room temperature. The resulting dark black solution was ultracentrifuged at 18,000g for 3 hours, followed by 120,000g for 4 hours and the resulting supernatant was separated to obtain EW-SWNT dispersion.

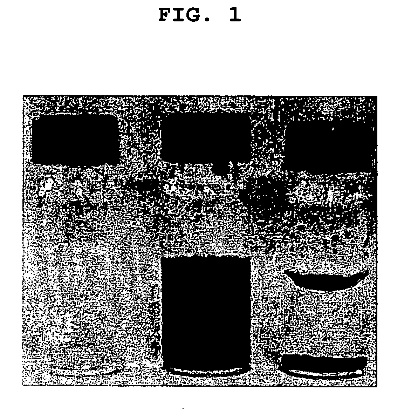

Fig. 1 shows photographs of vials containing (a) an aqueous solution of EW protein, (b) the inventive EW-SWNT dispersion and (c) a dispersion of only SWNTs in water. It can be clearly seen that the EW-SWNT dispersion is a dark homogeneous solution, whereas, the SWNTs dispersed simply in water are precipitated at the bottom.

It is well known that individually separated SWNTs exhibit sharp absorption peaks in visible and infrared spectra due to the van-Hove transitions of metallic and semiconducting SWNTs, while aggregated or bundled SWNTs exhibit broad and weak absorption peak.

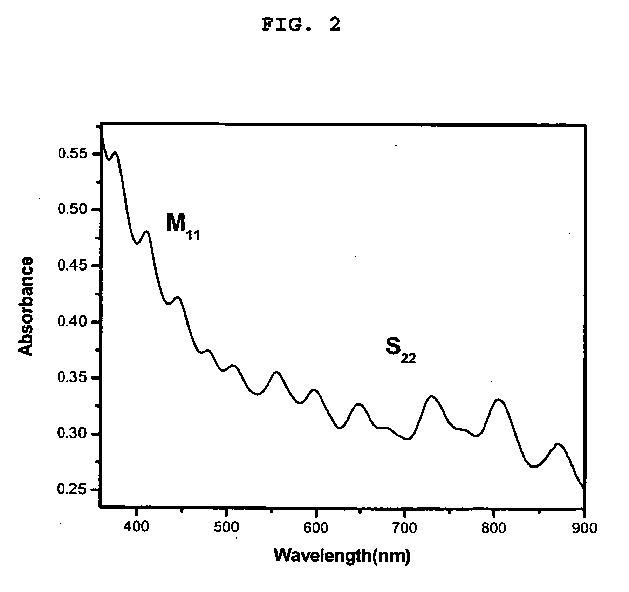

Fig. 2 shows UV/VIS absorbance spectra of the EW-SWNT in aqueous dispersion. The result clearly shows well-resolved peaks. The peaks centered from 440 nm to 600 nm and 550 nm to 800 nm are attributed to the first van Hove transition of metallic SWNTs (M₁₁), and the second van Hove transition of semiconducting SWNTs(S₂₂), respectively. From the absorbance spectra it was estimated that more than 20 mg/L of individually isolated nanotubes are presented in the dispersion, although this value varies slightly depending on the pH.

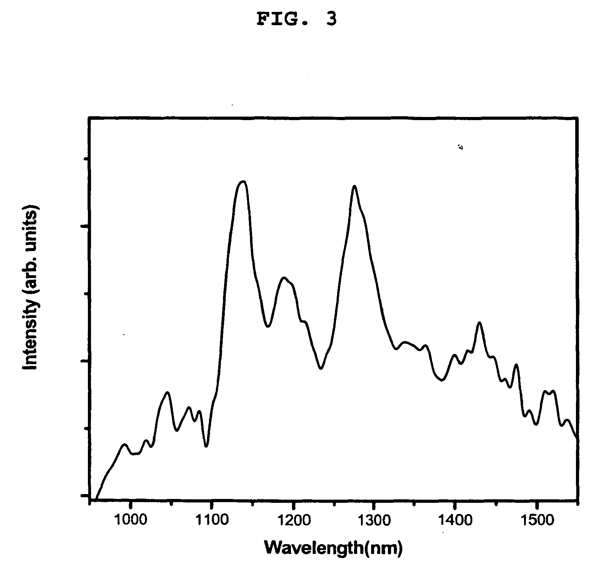

Further, the individually isolated nanotubes display near-infrared fluorescence, and thus, the dispersion was subjected to near-infrared photoluminescence spectroscopy (excitation: 523 nm). The emission spectrum of the EW-SWNT dispersion shows a typical emission band ranging from 900 nm to 1400 nm which corresponds to the S₁₁ transition (Fig. 3). This emission spectrum is a key evidence for the presence of individually separated nanotubes, since for bundled SWNTs, the interaction between the adjacent SWNTs quenches the luminescence.

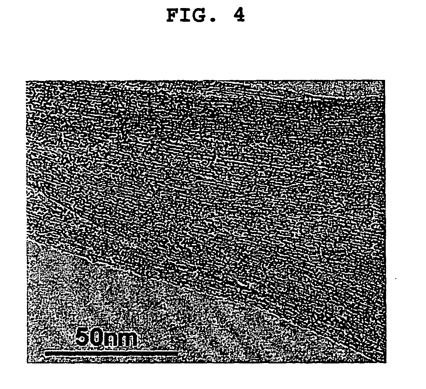

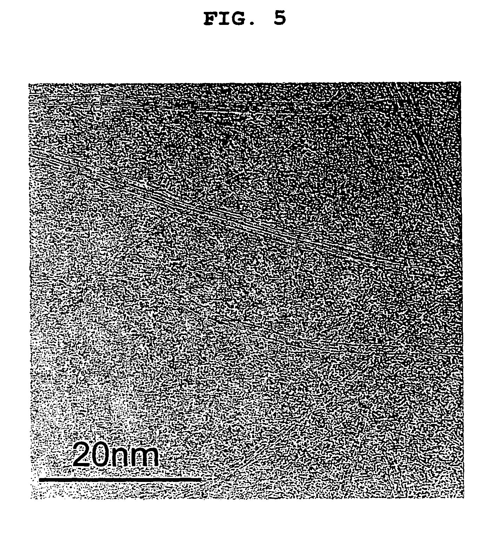

Furthermore, the morphology of the nanotubes in the SWNTs dispersion (Fig. 4) and EW-SWNT dispersion (Fig. 5) were analyzed by transmission electron microscopy. Fig. 5 shows the result that the nanotubes are highly dispersed and more than 50 % of the nanotubes were individually separated, although small bundles of 2-5 nanotubes also exist in the product. This result further verifies that the EW protein helps the nanotubes to remain individually in the aqueous dispersion due to the strong affinity between the EW protein and surface of the nanotubes.

Accordingly, the EW-SWNT of the present invention provides an aqueous dispersion of individually isolated SWNTs having non-covalent bond with the EW protein and it can be used in various fields in which well-preserved properties of isolated single-wall carbon nanotubes are required.

While the invention has been described with respect to the above specific embodiments, it should be recognized that various modifications and changes may be made to the invention by those skilled in the art which also fall within the scope of the invention as defined by the appended claims.

## Claims

1. A single-wall carbon nanotube-egg white protein composite comprising a single-wall carbon nanotube having non-covalent bonds with egg white protein.

2. An aqueous dispersion comprising the composite of claim 1.

3. A method for debundlizing single-wall carbon nanotubes comprising mixing the single-wall carbon nanotubes and egg white protein in an aqueous solution.

4. The method of claim 3, further comprising homogenizing the mixture of single-wall carbon nanotubes and egg white protein aqueous solution, and removing solid bodies from the resulting homogenate.

5. The method of claim 4, wherein the homogenization is conducted by ultrasonication.

6. The method of claim 4, wherein the solid bodies are removed by ultracentrifugation.

7. The method of claim 4, wherein carbon nanotubes having a length in the range of 500 nm to 2 µm are used in an amount of 3-10 weight % based on the amount of the egg white protein.

8. A device comprising the single-wall carbon nanotube-egg white protein composite of claim 1 or the aqueous dispersion of claim 2.

## Patentansprüche

1. Einwandige Kohlenstoffnanoröhre-Eiweißprotein-Verbund umfassend eine einwandige Kohlenstoffnanoröhre, die nicht-kovalente Bindungen mit Eiweißprotein aufweist.

2. Wässrige Dispersion umfassend den Verbund gemäß Anspruch 1.

3. Verfahren zum Entbündeln einwandiger Kohlenstoffnanoröhren umfassend das Mischen der einwandigen Kohlenstoffnanoröhren und Eiweißprotein in einer wässrigen Lösung.

4. Verfahren gemäß Anspruch 3, ferner umfassend das Homogenisieren des Gemisches aus einwandigen Kohlenstoffnanoröhren und der wässrigen Eiweißproteinlösung, und Entfernen der Festkörper aus dem entstandenen Homogenisat.

5. Verfahren gemäß Anspruch 4, **dadurch gekennzeichnet, dass** die Homogenisierung durchgeführt wird durch Ultraschallbehandlung.

6. Verfahren gemäß Anspruch 4, **dadurch gekennzeichnet, dass** die Festkörper entfernt werden durch Ultrazentrifugation.

7. Verfahren gemäß Anspruch 4, **dadurch gekennzeichnet, dass** die Kohlenstoffnanoröhren eine Länge im Bereich von 500 nm bis 2 µm aufweisen und verwendet werden in einer Menge von 3-10 Gewichtsprozent basierend auf der Menge von dem Eiweißprotein.

8. Vorrichtung umfassend den einwandige Kohlenstoffnanoröhre-Eiweißprotein-Verbund gemäß Anspruch 1 oder die wässrige Dispersion gemäß Anspruch 2.

## Revendications

1. Composite à base de protéine de blanc d'oeuf et de nanotubes de carbone monoparoi comprenant des nanotubes de carbone monoparoi ayant des liaisons non covalentes avec une protéine de blanc d'oeuf.

2. Dispersion aqueuse comprenant le composite de la revendication 1.

3. Procédé pour disperser et séparer les nanotubes de carbone monoparoi comprenant l'étape consistant à mélanger les nanotubes de carbone monoparoi et la protéine de blanc d'oeuf dans une solution aqueuse.

4. Procédé selon la revendication 3, comprenant en outre l'étape consistant à homogénéiser le mélange de nanotubes de carbone monoparoi et de protéine de blanc d'oeuf en solution aqueuse, et l'étape consistant à retirer les corps solides du broyat résultant.

5. Procédé selon la revendication 4, dans lequel l'homogénéisation est réalisée par un procédé ultrasonique.

6. Procédé selon la revendication 4, dans lequel les corps solides sont retirés par ultracentrifugation.

7. Procédé selon la revendication 4, dans lequel des nanotubes de carbone ayant une longueur comprise dans un intervalle allant de 500 nm à 2 µm sont utilisés dans une quantité de 3 à 10 % en masse par rapport à la quantité de protéine de blanc d'oeuf.

8. Dispositif comprenant le composite à base de protéine de blanc d'oeuf et de nanotubes de carbone monoparoi de la revendication 1 ou la dispersion aqueuse de la revendication 2.
